# EUROPEAN PATENT APPLICATION

(11) **EP 3 831 363 A1**
(43) Date of publication of application: **09.06.2021**
(21) Application number: 20211060.7
(22) Date of filing: 01.12.2020
(51) Int. Cl.: A61K 9/00, A61K 9/12, A61K 31/465, A61P 25/34, A61P 29/00

(54) **(S)-3-[1-METHYLPYRROLIDIN-2-YL]PYRIDINE, ANALOGUES THEREOF, PRECURSORS THEREOF, OR ITS DERIVATIVES, FOR THE USE AS A PHARMACEUTICAL IN FORM OF AN AEROSOL AND DISPENSING DEVICE**

(30) Priority: 02.12.2019 LU 101510
(71) Applicant: Solis Herrera, Arturo, CP20000 Aguascalientes (MX)
(72) Inventor: Solis Herrera, Arturo, CP20000 Aguascalientes (MX)
(74) Representative: Patentanwälte Bals & Vogel

(57) **Abstract**

The invention relates to a (S)-3-[1-Methylpyrrolidin-2-yl]pyridine, analogues thereof, precursors thereof, or its derivatives, for the use as a pharmaceutical in form of a substance as a colloid of fine solid and/or liquid particles in a fluid.

According to the invention the substance is an aerosol.

## Description

The present invention relates to a (S)-3-[1-Methylpyrrolidin-2-yl]pyridine, analogues thereof, precursors thereof, or its derivatives - containing pharmaceutical in form of an aerosol. In addition the invention relates to a dispensing device for the use with a drug delivery system.

### Field of invention

(S)-3-[1-Methylpyrrolidin-2-yl]pyridine, analogues thereof, precursors thereof, or its derivatives, also known as nicotine, is an alkaloid found predominantly in plants like tobacco and cocoa and in lower quantities even in plants like tomato, potato, eggplant and green pepper, all belonging to the nightshade family of plants (Solanaceae). (S)-3-[1-Methylpyrrolidin-2-yl]pyridine is accumulated in the leaves of these plants while its biosynthesis is taken place in the particular roots. Thereby, (S)-3-[1-Methylpyrrolidin-2-yl]pyridine constitutes approximately 0,6 to 0,3% of the dry weight of for example the tobacco plant and is present in the range of 2 to 7 µg/KG of various edible plants.

Further, (S)-3-[1-Methylpyrrolidin-2-yl]pyridine is a stimulant drug acting as an agonist to most nicotinic acetylcholine receptors (nAChR). In lesser doses of approximately 1 mg the substance adds on as a stimulant in mammals, while high amounts of approximately 50 to 100 mg can start being harmful.

(S)-3-[1-Methylpyrrolidin-2-yl]pyridine is hygroscopic, oily, colorless or pale yellow liquid, which is miscible with water and in its base form comprises a pyridine odor. (S)-3-[1-Methylpyrrolidin-2-yl]pyridine comprises a molecular weight of approximately 172 g/mol, is readily soluble in alcohol, ether or light petroleum. Further, (S)-3-[1-Methylpyrrolidin-2-yl]pyridine forms salts with acids that are solid and water soluble. The substance is further absorbed from the respiratory tract, intact skin, the gastro-intestinal tract and by the mucosa. Thereby, (S)-3-[1-Methylpyrrolidin-2-yl]pyridine undergoes extensive first pass metabolism when administered orally and/or nasally. Furthermore, (S)-3-[1-Methylpyrrolidin-2-yl]pyridine easily penetrates the skin, particularly the mucosa. The substance is able of being quickly distributed throughout the bloodstream and is even able to cross the blood-brain-barrier. The half-life of (S)-3-[1-Methylpyrrolidin-2-yl]pyridine in the body is approximately two hours.

S)-3-[1-Methylpyrrolidin-2-yl]pyridine mentioned within the scope of the invention is always understood as (S)-3-[1-Methylpyrrolidin-2-yl]pyridine itself, analogues thereof, precursors thereof, its derivatives or nicotine which mimic the effect of (S)-3-[1-Methylpyrrolidin-2-yl]pyridine, either alone or in combination with other active substances can be used.

### Background of the invention

In US 4,839,174 a controlled release transdermal delivery system for nicotine administration is disclosed. The system comprises an impermeable backing layer, a polyurethane matrix layer with about 5 to 50% of nicotine and an adhesive skin-contacting layer. The system is designated to administer nicotine for smoking cessation therapy with uses of a period of 24 hours and more. The disadvantage of such a delivery system is that due to natural movements and actions the device is not reliably sticking to the skin of the user and thereby the substance cannot reliably be absorbed by the individual.

### Detailed description of the invention

This problem is solved according to the invention by all features of claim 1. The depending claims specify embodiments. Further, this problem is also solved by all features of claim 11 wherein the dependent claims specify possible embodiments.

The special benefit of this invention is that a certain dosage of (S)-3-[1-Methylpyrrolidin-2-yl]pyridine, analogues thereof, precursors thereof or its derivatives, can be administered and reliably delivered to the desired destination within the body.

Advantageously, an aerosol can contain divers concentrations of (S)-3-[1-Methylpyrrolidin-2-yl]pyridine, analogues thereof, precursors thereof or its derivatives, in order to be absorbed by the body. The aerosol thereby comprises discrete particles of (S)-3-[1-Methylpyrrolidin-2-yl]pyridine. Generally, aerosols vary in their disparity. Most aerosols are polydisperse colloidal systems comprising a whole range of particle sizes. On the other hand also monodisperse aerosols exist and contain particles of uniform sizes. Advantageously, these particles are of a size, which makes them capable of being transported through a certain barrier of the body, particularly the skin and the mucosa and is even able to cross the blood-brain barrier.

In one preferred embodiment of the invention (S)-3-[1-Methylpyrrolidin-2-yl]pyridine is intended for inhalation. In general, inhalants are a broad range of drugs, whose volatile vapors or gases are taken in via the nose and/or trachea. Advantageously, inhalants are evenly distributed in a certain area and can be absorbed by the body, particularly the skin or mucosa in a distinct manner. The volatile substances are thereby evenly distributed in the certain area they are applied to and penetrate the skin in a defined way. Preferably the inhalants are taken in by room temperature of approx. 18 to 22°C.

In one possible embodiment of the invention (S)-3-[1-Methylpyrrolidin-2-yl]pyridine, analogues thereof, precursors thereof or its derivatives, is intended for oral and/or nasal administration. Alternatively or additionally, the substance is also intended for nasal administration. In general aerosols are applied to the oral and/or nasal cavity and/or to the respiratory tract of an individual. Hereby, an aerosol containing particles of (S)-3-[1-Methylpyrrolidin-2-yl]pyridine is administered to the oral and/or nasal cavity of the individual so that it is capable of being inhaled. Therefore, the particles are of an adequate size for inhalation. By administration the oral and/or nasal and/or respiratory tract are moistened with the substance. Thereby the aerosol can be a liquid spray, which contains the substance in a liquid manner, particularly an inert liquid carrier. Said adequate size of particles regards to liquid and/or solid components within the aerosol, which in particular comprise dust-like sizes. However, it is provided that the sizes are valid for the migration through the oral and/or nasal cavity into the respiratory tract. A preferred aerosol size is thereby approximately 1 µm to approximately 15 µm in diameter. Preferably, the size is between approx. 5 µm to approx. 10 µm in diameter. In a further preferred embodiment of the invention it can be beneficial if an emulsifying agent is added. Preferably, fatty acids can be used as an emulsifying agent, particularly lecithin in order to reduce the surface tension of the aerosol and thereby enhances smaller particle sizes for an improved penetration trough at the target destination.

Advantageously, (S)-3-[1-Methylpyrrolidin-2-yl]pyridine, analogues thereof, precursors thereof or its derivatives, is intended for the application on the skin, particularly on the mucosa. The skin is an organ of the integumentary system of mammals consisting of multiple layers epithelial tissues with underlying muscles and organs. Thereby, the mucosa is a particular kind of skin, so called mucous membrane. The mucosa is mostly of endodermal origin and is involved in absorption and recreation. The mucosa of the present invention particularly refers to the oral and/or nasal cavity and/or the respiratory tract. For the skin the stratum corneum constitutes the skin barrier for the diffusion of substances. Herein, the application of (S)-3-[1-Methylpyrrolidin-2-yl]pyridine as a substance applied to the skin is able to transdermal penetrate the stratum corneum. Preferably, the solution is thereby applied as an aerosol. It can also be applied as a spray, a roll-on or another spreading device. The application of the substances to the skin allows an uptake into the bloodstream where the substance can affect the organism and thereby reduce the symptoms of diverse diseases. It is an advantage of an application to the skin that no digestion is required and the effective substance can be applied in the vicinity of the target tissue. The substance is directly transferred via the bloodstream to the predetermined pharmacological target location. Moreover, an application to the skin is fast drying, quickly absorbed and non-irritating. Further, it is invisible after application and a reliable intake occurs. Further, applying a spray to the outer skin is an advantage for individuals needing a repeated administration of the substance since a spray can be applied locally without any effort of administering.

Furthermore, it can be provided that (S)-3-[1-Methylpyrrolidin-2-yl]pyridine, analogues thereof, precursors thereof or its derivatives, contain a vehicle substance, particularly distilled water. The present delivery system contains a water-based aerosol formulation. For oral preparations (S)-3-[1-Methylpyrrolidin-2-yl]pyridine can be used alone or in combination with appropriate additives, such as lactose, mannitol, corn starch or potato starch, with binders, such as crystalline cellulose, cellulose derivatives, acacia, corn starch or gelatins, with disintegrators, such as corn starch, potato starch or sodium carboxymethylcellulose, with lubricants, such as talc or magnesium stearate, and if desired, with diluents, buffering agents, moistening agents, preservatives and flavoring agents. Thereby, (S)-3-[1-Methylpyrrolidin-2-yl]pyridine is a formulation, which is soluble in or miscible with a liquid carrier. Another advantage is that the substance is soluble or miscible at room temperature. Alternatively and/or additionally, (S)-3-[1-Methylpyrrolidin-2-yl]pyridine may be in form of a salt, which is solid at room temperature but can be dissolved in the liquid carrier, particularly in water. Therefore, (S)-3-[1-Methylpyrrolidin-2-yl]pyridine like its salt formulations are generally soluble in water. A process of mixing can occur in order to generate a homogeneous liquid mixture. Beneath (S)-3-[1-Methylpyrrolidin-2-yl]pyridine also pharmacological analogues or derivatives or substances which mimic the effect of (S)-3-[1-Methylpyrrolidin-2-yl]pyridine, either alone or in combination with other active substances can be used. In order to produce a substance for a pharmacological treatment with an aerosol a combination of (S)-3-[1-Methylpyrrolidin-2-yl]pyridine, a sugar and a liquid carrier preferably consisting of water and/or alcohol is used in order to generate (S)-3-[1-Methylpyrrolidin-2-yl]pyridine suitable for the delivery to the oral and/or nasal cavity and/or the respiratory tract. As another additive lactose can be used, which is also soluble in a liquid carrier. The preferred liquid carrier is water. Other liquid carriers can also be used, for example alcohols, preferably in combination with water. Preferably, the alcohol is a primary alcohol. Further, the alcohol is preferably a lower alkyl alcohol. Particularly, the alcohol can be ethanol, isopropanol, ethylacetate, chloroform and/or acetone. Within the liquid carrier alcohol serves as a solvent. When alcohol is added the preferred ratio of alcohol to water in the liquid carrier is approximately about 1:1 to 1:10, preferably about 1:2 to 1:8 and more preferably about 1:5 to 1:7 parts. In addition to alcohols like ethanol also glycerol, propylene glycol can be used. Advantageously, propellants can be used in order to apply the aerosol as a spray. It can comprise a propellant, which is stored in a pressurized container. As a propellant preferably carbons, propan, butan, dimethylether, nitrogen or air can be used.

In one special embodiment of the invention (S)-3-[1-Methylpyrrolidin-2-yl]pyridine, analogues thereof, precursors thereof or its derivatives comprise a concentration of 0,3 mg/ml to 4,5 mg/ml, preferably 1,5 mg/ml to 3,0 mg/ml. Thereby, propellants are not considered as part of the active solution. The concentration of the active agent further depends on the liquidity at skin temperature (approximately 32°C) and/or the temperature of the oral and/or nasal cavity (approximately 32°C). Herein, the delivery system applies a dose of the active agent of about 0,3 mg/ml to 4,5 mg/ml per unit dose. Preferably, 1,5 mg/ml to 3,0 mg/ml is applied. Advantageously, the applied concentration of (S)-3-[1-Methylpyrrolidin-2-yl]pyridine is not toxic. Using a cytotoxic compound can result in a variety of cell fates. The appearance of cytotoxic concentrations can result in necrosis (loss of membrane integrity) and thereby cell lysis, stopping cell growth and dividing or even apoptosis. In order to investigate the cytotoxicity level of certain substances cytotoxicity assays are widely used in the pharmaceutical industry. Further indicators being investigated in order to determine the level of cytotoxicity are for example TNFα (tumor necrosis factor alpha), IFNγ (Interferon gamma) or IL-6 (interleukin). TNFα is a cell signaling protein mainly involved in systemic inflammation. Cells that produce TNFα are mainly macrophages, lymphocytes, mast cells, or neurons. However, the main function of TNFα is in the regulation of immune cells. Further, IFNγ is a soluble cytokine. IFNγ is a cytokine that is critical for immunity against viral, bacterial and protozoal infections. Thereby it is an important activator of macrophages and inducer of MHC (major histocompatibility complex) expression. IFNγ has antiviral, immunoregulatory and anti-tumor properties. Further IL-6 is an interleukin that acts as both a pro-inflammatory cytokine and an anti-inflammatory myokine. In mammalian IL-6 is secreted by T-cells and macrophages to stimulate an immune response.

Advantageously, (S)-3-[1-Methylpyrrolidin-2-yl]pyridine, analogues thereof, precursors thereof or its derivatives, triggers stimulation of cholinergic receptors, particularly nicotinic receptors. Nicotine acetylcholine receptors (nAChR) are neuronal receptor proteins that signal upon a chemical stimulus. The cholinergic receptors are forming ligand gated ion channels in the plasma membrane of the certain neuron. They are expressed on the presynaptic and postsynaptic side of the neuro muscular junctions. As neurotropic receptors they are directly linked to ion channels and thereby are not dependent on second messengers. Neuron receptors are found in the central nervous system as well as in the peripheral nervous system of mammalians. Generally, ligand gated ion channels require the binding of a chemical messenger for opening. The endogenous agonist is acetylcholine wherein they are also triggered by (S)-3-[1-Methylpyrrolidin-2-yl]pyridine, analogues thereof, precursors thereof or its derivatives. The action that (S)-3-[1-Methylpyrrolidin-2-yl]pyridine binds to nicotine cholinergic receptors can be regarded as a key energy source of the cell, as this is responsible for upregulating of energy. All nicotine cholinergic receptors produce excitatory postsynaptic effects. Thereby, energy is upregulated previous to an excitatory postsynaptic effect. Further, the administration of (S)-3-[1-Methylpyrrolidin-2-yl]pyridine upregulates the release of endogenous alpha-MSH. The melanocytes stimulating hormone (MSH) belong to peptide hormones that are produced in the central nervous system. These hormones stimulate the production inducing melanin by melanocytes in the skin and in the hair. Alpha-MSH induces multiple responses in nearly all cells of mammalians, thereby significantly improving and also maintaining good health conditions. Alpha-MSH is further related to chemical energy levels within the mammalian body and thereby fundamental for an optimal body performance.

In one advantageous embodiment of the invention is (S)-3-[1-Methylpyrrolidin-2-yl]pyridine, analogues thereof, precursors thereof or its derivatives is used for the treatment of diseases related to pain. Further, these substances also modulate many disease pathologies like skin physiology, melanocytes function, nerve regeneration, behavior and learning as well as memory, obesity and energy metabolism, brain inflammation, autoimmune diseases, septic shock, endotoxemia, renal ischemic occlusion and reperfusion, mesenteric occlusion and reperfusion, diabetes, viral related diseases like H1N1 intoxications, viral related intoxications like Ebola, congenital mal formations and cancer as well as inflammatory diseases. Particularly, the substance is related to diseases as joint pain and further to diseases related to nerves as well as to skin. Mainly, these diseases can be treated in mammalians preferably in humans and/or animals.

Advantageously, (S)-3-[1-Methylpyrrolidin-2-yl]pyridine, analogues thereof, precursors thereof or its derivatives, are administered every 24 hours. Preferably, the substance is administered every 12 hours, preferably every 6 hours. Thereby, the frequency of administration depends upon the variety of disease. In administration every 2 to 3 hours is likewise possible.

The present invention further relates to a dispensing device for use with a drug delivery system. Thereby, the dispensing device comprises a housing with an air inlet means and a nozzle through which a dosage of pharmacological substance entrained in a fluid flow. Further, having means for actuating said drug delivery system to dispense a measured dosage of pharmacological relevant substances. The dispensing device is thereby suitable for applying (S)-3-[1-Methylpyrrolidin-2-yl]pyridine, analogues thereof, precursors thereof or its derivatives. The dispensing device is appropriate for transdermal delivery of a volatile active substance, preferably (S)-3-[1-Methylpyrrolidin-2-yl]pyridine to an area of skin comprising a container. Within the container a solution is stored containing the substance, which is dissolved in a liquid carrier. An applicator nozzle for delivering a spray of the substance onto the skin is further provided. Preferably, a spacer extending from the housing for placement against the skin to stabilize the nozzle in a spaced relationship to the area of the skin is intended. The dispenser is also appropriate for an application to the mucosa by an application through inhalation. Thereby, the dispenser is placed within the front portion of the mouth or the nose. Preferably, an activation of the dispenser occurs along with an inhalation process. Moreover, the dispensing device particularly dispenses the pharmacological relevant substance, particularly (S)-3-[1-Methylpyrrolidin-2-yl]pyridine in single dosages. The system typically applies the dosage over an area, particularly of skin and/or mucosa of at least 5 m², preferably no more than about 100 m². Preferably, the dose will be applied of an area of skin of approximately 20 cm².

The present invention is further described in detail with respect to the accompanying examples and figures. Features discussed with the description of the examples and the figures can be freely combined with each other. The features mentioned in the claims and specifications are essential to the invention, either in themselves or in given combination.

### Examples and Figures

In an example a treatment with (S)-3-[1-Methylpyrrolidin-2-yl]pyridine of viral diseases like for example H1N1 was tested on chicken, infected with the H1N1 virus. Therefore, 30000 diseased chicken were divided into two groups of 15000. One group was treated with (S)-3-[1-Methylpyrrolidin-2-yl]pyridine at doses of 0,002 mg/kg each 24 hours. The other group represented the control group. 24 hours after treatment the treated group began to lay off eggs in contrary to the control group, which turned down the production of eggs. The treatment against intoxication was tested related to mercury, CN, cadmium, arsenic, herbicides, pesticides. As an example mercury poisoning induces a generalized failure, typical to low levels of energy, so that the mortality level is high. As a result 6 in 8 rats died at the dose of 4,5 mg/kg of mercury (HgHCI). In any of the organs edema and hemorrhage was examined. After a treatment of 0,0937 mg/ml with (S)-3-[1-Methylpyrrolidin-2-yl]pyridine mortality was reduced to 0,625 of 8 rats.

Figures
- Fig. 1: a diagram showing a treatment of a group of humans (n = 10/each) ulcer with (S)-3-[1-Methylpyrrolidin-2-yl]pyridine
- Fig. 2a: a diagram showing a cytotoxicity test with WI-38 cells treated with (S)-3-[1-Methylpyrrolidin-2-yl]pyridine
- Fig. 2b: a diagram showing a cytotoxicity test with A549 cells treated with (S)-3-[1-Methylpyrrolidin-2-yl]pyridine
- Fig. 2c: a diagram showing a cytotoxicity test with HS683 cells treated with (S)-3-[1-Methylpyrrolidin-2-yl]pyridine
- Fig. 3: a diagram showing a cytotoxicity test of human PBLs treated with (S)-3-[1-Methylpyrrolidin-2-yl]pyridine
- Fig. 4a: a diagram showing the influence of (S)-3-[1-Methylpyrrolidin-2-yl]pyridine on the proliferation of WI-38 cells
- Fig. 4b: a diagram showing the influence of (S)-3-[1-Methylpyrrolidin-2-yl]pyridine on the proliferation of A549 cells
- Fig. 4c: a diagram showing the influence of (S)-3-[1-Methylpyrrolidin-2-yl]pyridine on the proliferation of HS683 cells
- Fig. 5a: a diagram showing the effect of (S)-3-[1-Methylpyrrolidin-2-yl]pyridine on TNF-α production
- Fig. 5b: a diagram showing the effect of (S)-3-[1-Methylpyrrolidin-2-yl]pyridine on IFN-γ production

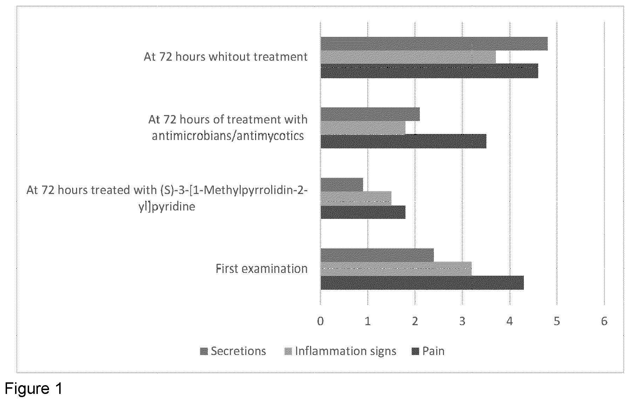

In a further example (Fig. 1) the effect of (S)-3-[1-Methylpyrrolidin-2-yl]pyridine is shown. Thereby the healing process of a human ulcer is examined as a basic condition. Different ulcers (n = 10 for each group) are examined each after 72 h treatment with (S)-3-[1-Methylpyrrolidin-2-yl]pyridine (Group A), treatment with antimicrobians/antimycotics (group B) and without treatment (group C). As a result the treatment with (S)-3-[1-Methylpyrrolidin-2-yl]pyridine resulted in an increased healing effect (group A) compared to the treatment with antimicrobians/antimycotics (group B) and without treatment (group C) (Fig. 1).

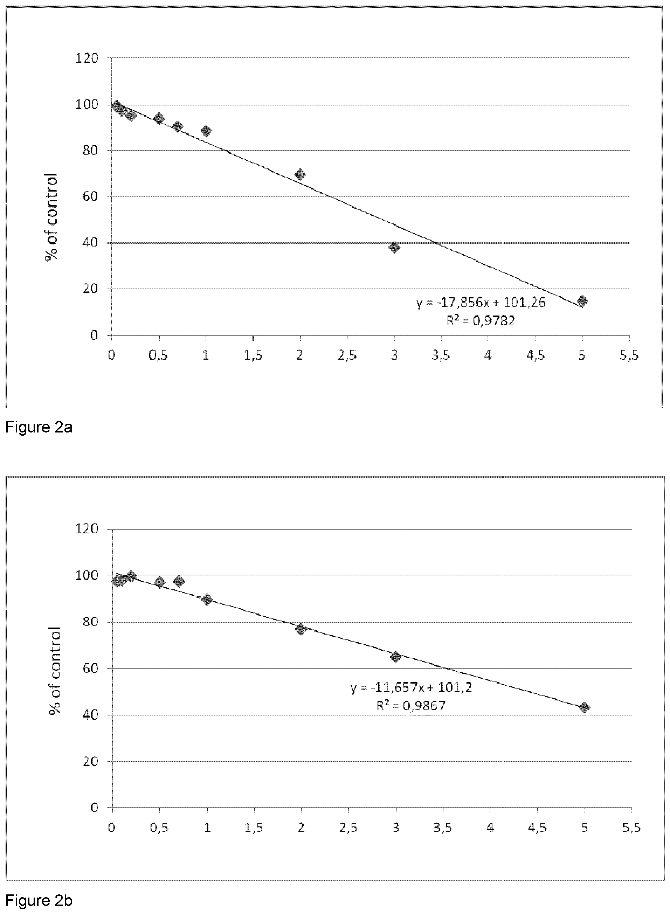

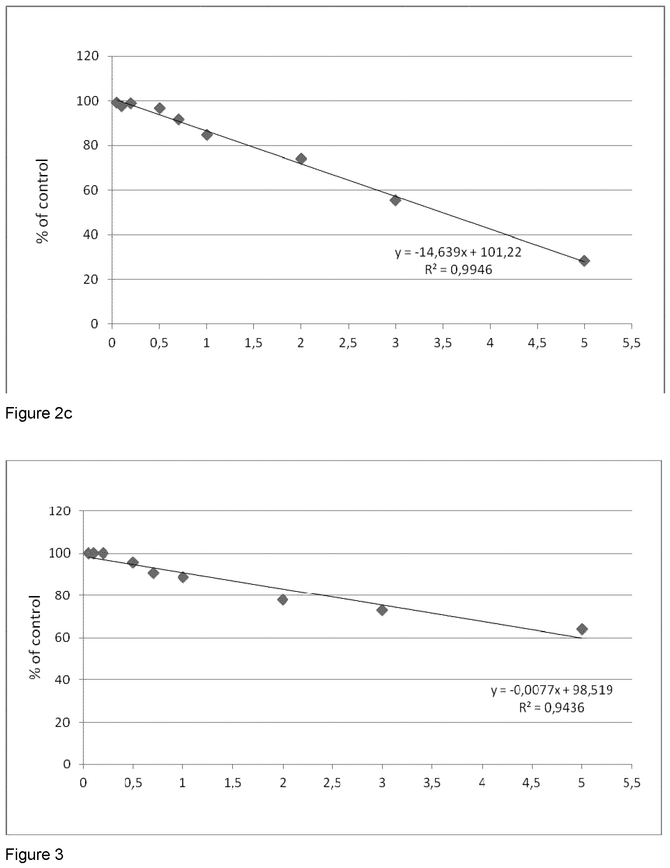

In a further example cytotoxicity tests were performed with three different cell lines (Fig. 2a, 2b, 2c):
- WI-38 (human diploid cell line from normal embryonic lung tissue)
- A549 (human lung adenocarcinoma epithelial cell line) and
- HS683 (human neuronal glioma cell line).

Thereby, cell viability was determined on the basis of the mitochondria-dependent reduction of MTT (3-[4,5-dimethyltiazol 2yl]-2,5-diphenyltetrazolium bromide) to formazan. Cell lines (2x10⁵cell/ml) as well as leukocytes (2x10⁶ cells/ml) were cultured in 96-well plates for 48 hours in the presence of various concentrations of (S)-3-[1-Methylpyrrolidin-2-yl]pyridine (5-0,05 mg/ml). Next cells were treated with 20 µl of MTT. After 3 hours of incubation at 37°C, the formazan blue formed in the cells was dissolved in 100 µl of SDS (sodium dodecylsulfate) for 3 hours in 37°C. The optical density was measured at 570 nm.

After the cytotoxicity tests LC₅₀ (LCso-lethal concentration, 50%-dose required to kill half of the cells after a specified test duration) were estimated for each cell lines and PBLs (leukocytes of peripheral blood). Additionally for PBLs LC₁₀ and LC₅ were calculated. On the basis of the results for leukocytes three different concentrations of (S)-3-[1-Methylpyrrolidin-2-yl]pyridine were selected for evaluation of the effect of cytokine production.

Further, a proliferation test was prepared for the same three cell lines: WI-38, A549 and HS 683. 3x10⁴ WI-38 cells and 2x10⁴ A549 and HS 683. Cells were seeded into each wells in 96-well culture plates in appropriate culture medium with 10% FBS (fetal bovine serum). After 24 h incubation in 37°C/5%CO₂ medium were removed and replaced with tested concentrations of (S)-3-[1-Methylpyrrolidin-2-yl]pyridine in culture medium. After 96 hours incubation in 37°C/5%CO₂ MTT assay was performed for establishing the cell viability.

Studies of the effect of (S)-3-[1-Methylpyrrolidin-2-yl]pyridine on cytokine production was performed on PBLs isolated ex vivo from 10 healthy volunteers (male 5; female 5; age 23-45). Next, leukocytes were treated with selected, nontoxic concentrations of (S)-3-[1-Methylpyrrolidin-2-yl]pyridine (1 mg/ml; 500 µg/ml, 300 µg/ml) without or with 5 µg/ml of LPS (lipopolysaccharide). After 24 hours of incubation in 37°C/5%CO₂ samples of leukocytes and supernatants were collected. For evaluation of the influence of (S)-3-[1-Methylpyrrolidin-2-yl]pyridine on IFN-γ (interferon) production additionally samples were collected after 72 hours of incubation.

Cytokine levels were determined using ELISA kits (BD Biosciences, USA) for human IFNγ, TNF-α and IL-6 and DuoSet for human IL-3 (R&D Systems, USA) according to the producers' instructions. The optical density was measured at 450 nm using a Multiskan RC spectrophotometric reader (Thermo Labsystems, USA), Cytokine concentrations were expressed in pg/ml.

In summary the LC₅₀ of (S)-3-[1-Methylpyrrolidin-2-yl]pyridine for a cell line WI-38 is 3,0 mg/ml, for the cell line A549 is 4,5 mg/ml and for the cell line HS-683 is 3,5 mg/ml.

In Figure 3 the results of the cytotoxicity test of human PBLs is shown.

On the basis of the results of MTT assay for human leukocytes the LC₅₀, LC₁₀ and LC₅ were evaluated. The LC₅₀ is a concentration of 7 mg/ml (S)-3-[1-Methylpyrrolidin-2-yl]pyridine. The LC₁₀ is in a concentration of 1 mg/ml and LC₅ is in a concentration of 0,5 mg/ml (S)-3-[1-Methylpyrrolidin-2-yl]pyridine.

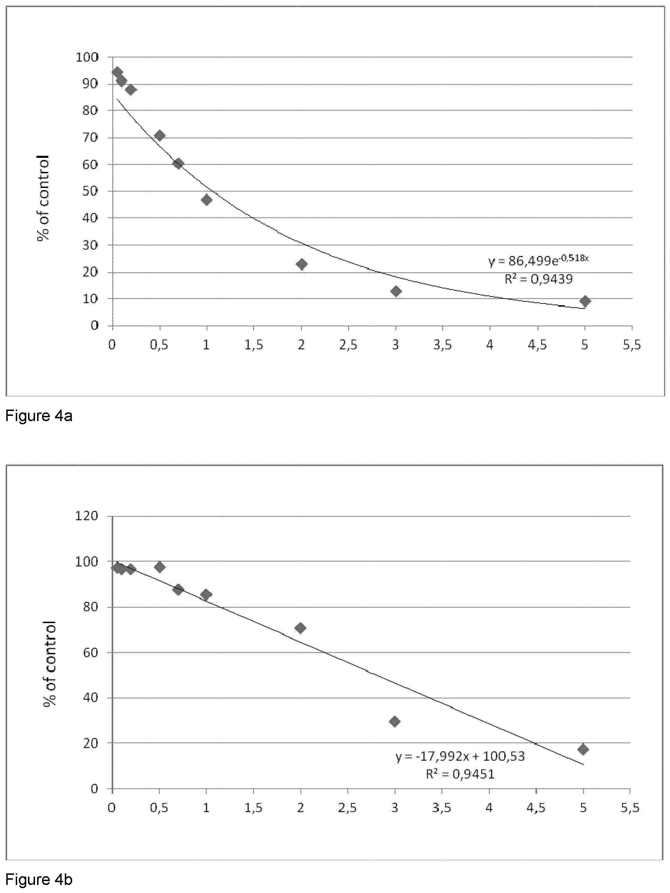

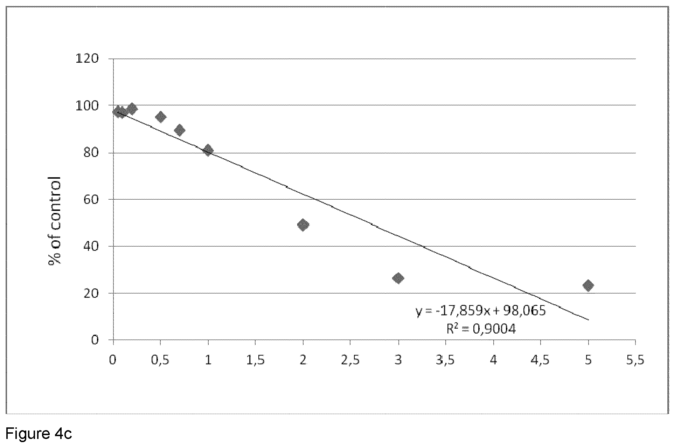

In a further example (Figures 4a to 4c) the influence of (S)-3-[1-Methylpyrrolidin-2-yl]pyridine on the proliferation of divers cell lines was investigated. Figure 3a shows the effect on WI-38 cells. For the cell line WI-38 1,0 mg/ml of (S)-3-[1-Methylpyrrolidin-2-yl]pyridine inhibits 50% of the proliferation of these cells, whereby 4,5 mg/ml inhibits a proliferation of 99%.

For the cell line A549 a concentration of 2,0 mg/ml (S)-3-[1-Methylpyrrolidin-2-yl]pyridine inhibits 50% of the proliferation whereas the concentration of 5,5 mg/ml inhibits 99% of the proliferation.

For the cell line HS-683 2,9 mg/ml (S)-3-[1-Methylpyrrolidin-2-yl]pyridine inhibits 50% of the proliferation of these cells wherein 99% of proliferation are inhibited in a concentration of 5,5 mg/ml.

In another example the cytokine production is evaluated utilizing the effect of (S)-3-[1-Methylpyrrolidin-2-yl]pyridine on TNF-α production. As shown in Figure 4a the preparation increases the level of cytokines in a dose-dependent manner. The effect was observed only for unstimulated human PBLs.

At the same time studies of the effect of (S)-3-[1-Methylpyrrolidin-2-yl]pyridine on IFN-γ production showed that the preparation strongly decreases the level of interferon in a dose-dependent manner. The effect was observed for LPS stimulated human PBLs. There were poor or no spontaneous IFN-γ production after 24 hours and 72 hours of incubation of PBLs. At the same time no effect of (S)-3-[1-Methylpyrrolidin-2-yl]pyridine was observed for IL-6 production by human PBLs.

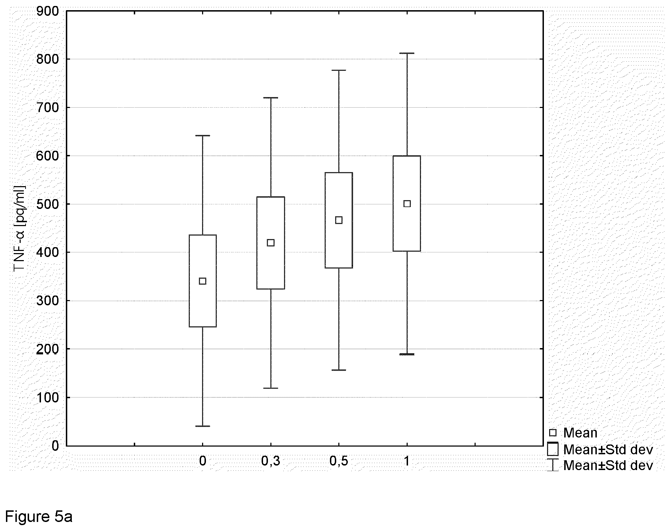

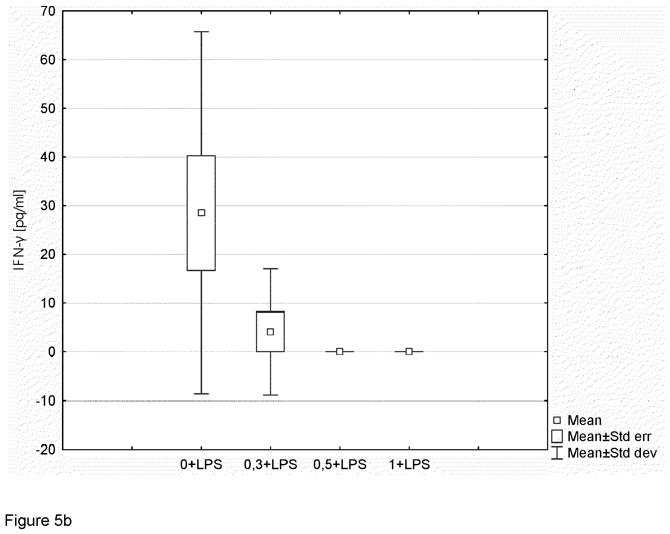

Shown in Figures 1 to 5 allow cytotoxicity of (S)-3-[1-Methylpyrrolidin-2-yl]pyridine was observed. The preparation inhibits 50% of cell proliferation in doses higher than 1 mg/ml. All other concentrations were harmless. Preparation increased TNF-α production by unstimulated human PBLs in a dose-dependent manner. Further, (S)-3-[1-Methylpyrrolidin-2-yl]pyridine strongly reduces IFN-γ production by LPS-stimulated human PBLs. The effect was again dose-dependent.

Aforesaid discussion of the different embodiments is only carried out by the way of example and does not limit the scope of the protection of the present invention.

## Claims

1. (S)-3-[1-Methylpyrrolidin-2-yl]pyridine analogues thereof, precursors thereof,
or its derivatives, for the use as a pharmaceutical in form of a substance as a colloid of fine solid and/or liquid particles in a fluid, **characterized in that** the substance is an aerosol.

2. (S)-3-[1-Methylpyrrolidin-2-yl]pyridine according to Claim 1, **characterized in that** the substance is intended for inhalation.

3. (S)-3-[1-Methylpyrrolidin-2-yl]pyridine according to Claim 2, **characterized in that** the substance is taken in by room temperature of approx. 18 to 22°C.

4. (S)-3-[1-Methylpyrrolidin-2-yl]pyridine analogues thereof, precursors thereof, or its derivatives, according to any one of Claims 1 to 3, **characterized in that** the substance is intended for oral administration.

5. (S)-3-[1-Methylpyrrolidin-2-yl]pyridine analogues thereof, precursors thereof, or its derivatives, according to Claim 4, **characterized in that** for oral preparations (S)-3-[1-Methylpyrrolidin-2-yl]pyridine is used alone or in combination with appropriate additives, such as lactose, mannitol, corn starch or potato starch, with binders, such as crystalline cellulose, cellulose derivatives, acacia, corn starch or gelatins, with disintegrators, such as corn starch, potato starch or sodium carboxymethylcellulose, with lubricants, such as talc or magnesium stearate, and if desired, with diluents, buffering agents, moistening agents, preservatives and flavoring agents.

6. (S)-3-[1-Methylpyrrolidin-2-yl]pyridine analogues thereof, precursors thereof, or its derivatives, according to one of Claim 1 to 3, **characterized in that** the substance is intended for nasal administration.

7. (S)-3-[1-Methylpyrrolidin-2-yl]pyridine analogues thereof, precursors thereof, or its derivatives, according to Claim 1 to 3, **characterized in that** the substance is intended for application on the skin, particularly on the mucosa.

8. (S)-3-[1-Methylpyrrolidin-2-yl]pyridine analogues thereof, precursors thereof, or its derivatives, according to one of Claims 1 to 7, **characterized in that** the fluid contains a vehicle substance, particularly water.

9. (S)-3-[1-Methylpyrrolidin-2-yl]pyridine analogues thereof, precursors thereof, or its derivatives, according to Claim 8, **characterized in that** the aerosol is a liquid spray, which contains the substance in an inert liquid carrier, particularly wherein a size of particles regards to liquid and/or solid components within the aerosol, wherein a preferred aerosol size is thereby approximately 1 µm to approximately 15 µm in diameter, preferably, the size is between approx. 5 µm to approx. 10 µm in diameter.

10. (S)-3-[1-Methylpyrrolidin-2-yl]pyridine analogues thereof, precursors thereof, or its derivatives, according to one of Claims 1 to 9, **characterized in that** the concentration of (S)-3-[1-Methylpyrrolidin-2-yl]pyridine is 0,3 mg/ml to 4,5 mg/ml, preferably 1,5 mg/ml to 3,0 mg/ml.

11. (S)-3-[1-Methylpyrrolidin-2-yl]pyridine analogues thereof, precursors thereof, or its derivatives, according to one of Claims 1 to 10, **characterized in that** a stimulation of cholinergic receptors, particularly nicotinic receptors, occurs.

12. (S)-3-[1-Methylpyrrolidin-2-yl]pyridine analogues thereof, precursors thereof, or its derivatives, according to one of Claims 1 to 11, for use in the treatment of diseases related to pain, particularly joint pain and/or nerves and/or skin, in humans and/or in animals, particularly in mammalians.

13. (S)-3-[1-Methylpyrrolidin-2-yl]pyridine analogues thereof, precursors thereof, or its derivatives, according to one of Claims 1 to 12, **characterized in that** the treatment is intended to administer every 24 hours, preferably every 12 hours, particularly preferably every 6 hours.

14. A dispensing device for use with a drug delivery system, wherein the dispensing device comprises a housing with an air inlet means and a nozzle through which a dose of medicament entrained in an fluid flow is, and having means for actuating said drug delivery system to dispense a measured dose of medicament, **characterized in that** (S)-3-[1-Methylpyrrolidin-2-yl]pyridine analogues thereof, precursors thereof, or its derivatives, according to one of Claims 1 to 13 is applied.

15. Dispensing device according to Claim 14, **characterized in that** the medicament is dispensed in a single dosage.
